# EUROPEAN PATENT SPECIFICATION

(11) **EP 0 413 706 B1**
(45) Date of publication and mention of the grant of the patent: **02.09.1998**
(21) Application number: 89904704.7
(22) Date of filing: 15.03.1989
(51) Int. Cl.: C07D 253/08, A61K 31/53

(54) **1,2,4-BENZOTRIAZINE OXIDES AS RADIOSENSITIZERS AND SELECTIVE CYTOTOXIC AGENTS**
1,2,4-BENZOTRIAZIN-N-OXIDDERIVATE ALS RADIOSENSIBILISIERENDE UND SELEKTIVE CYTOTOXISCHE MITTEL
OXYDES DE 1,2,4-BENZOTRIAZINE UTILISES COMME AGENTS RADIOSENSIBILISATEURS ET AGENTS CYTOTOXIQUES SELECTIFS

(30) Priority: 18.03.1988 US 169873
(43) Date of publication of application: 27.02.1991
(73) Proprietor: SRI INTERNATIONAL, Menlo Park, California 94025-3493 (US)
(72) Inventor: LEE, William, W., Palo Alto, CA 94303 (US); BROWN, J., Martin, Stanford, CA 94305 (US); GRANGE, Edward, W., Palo Alto, CA 94306 (US); MARTINEZ, Abelardo, P., San Jose, CA 95129 (US); TRACY, Michael, Palo Alto, CA 94303 (US)
(74) Representative: Goldin, Douglas Michael
(86) International application number: US8901037
(87) International publication number: WO8908647

(56) References cited:
- EP-A- 1 090
- WO-A-88/02366
- DE-A- 2 404 375
- FR-A- 2 322 140
- GB-A- 1 234 845
- US-A- 2 489 352
- US-A- 2 489 359
- US-A- 3 482 024
- 35th ann. meeting of radiation res. soc and 7th ann. meeting of North Am. Hyperthermia group, Feb. 21-26 1987, Atlanta, US, 1986, page 114, No. FD-4; M. Brown et.al.
- Biochemical Pharmacology, 35, no.19, October 1, 1986, pages 3417-3420 K.R.Laderoute et al.
- Radiotherapy and Oncology, 12(3), 1988 pages 209-218 E.M. Zeman et al.
- Tetrahedron, 38(12), 1982, 1793-1796, R.H. Atallah et al.
- The Journal of Organic Chemistry, Vol. 24, No. 6, 1959 (American Chemical Society, (US),) J. Jiu et al: 'Syntheses in the 1,2,4-benzotriazine series', see page 813 - page 818, see page 814, tables I,II; page 815 table IV
- Journal of American Chemical Society, Vol. 76, No. 18, 1954 (American Chemical Society, (US),) F J Wolf et al: 'Benzotriazines.II. Synthesis of 3-amino-7-halo-1,2,4-benzotriazine-1-oxides', see page 4611 - page 4613, see page 4612, table I
- Journal of American Chemical Society, Vol. 76, No. 13, 1954 (American Chemical Society, (US),) F J Wolf et al: 'Benzotriazines.I. A new series of compounds having antimalarial activity', see page 3551 - page 3553, see page 3552, table I
- Journal of Chromatography, Vol. 131, 1977 (Amsterdam) M S F Ross: 'Chromatographic analysis of azapropazone and related benzotriazines', see page 448 - page 452, see page 448, compound IV
- Chemical Abstracts, volume 106, no. 13, 30 March 1987, (Columbus, Ohio, US), E M Zeman et al: 'SR-4233: a new bioreductive agent with high selective toxicity for hypoxic mammalian cells', see page 29, abstract 96684v, & Int. J. Radiat. Oncol., Biol., Phys. 1986, 12(7), 1239-42

## Description

### Technical Field

The invention relates to cytotoxic agents and radiotherapy effective against hypoxic cells. Specifically, the invention relates to selectively killing tumor cells and to sensitizing tumor cells to radiation using 1,2,4-benzotriazine oxides.

### Background Art

Hypoxic cell radiosensitizers are compounds that selectively increase the sensitivity of hypoxic cells to destructive radiation. Cytotoxins which have enhanced activity under hypoxic conditions also provide a means for selective destruction of cells under low oxygen pressure. This specificity for hypoxic cells is important because it is tumors that are typically characterized by such cells. Virtually all tumors which are present as solid masses contain these cells, while normal cells generally have an adequate supply of oxygen. Accordingly, anti-tumor agents can be made selective for tumors by virtue of high activity under hypoxic conditions, and radiation can be employed more effectively in the presence of these sensitizers.

Of course, the use of radiation treatment to destroy tumor cells is only practical if damage to the surrounding normal tissue can be minimized or avoided. The effects of radiation are enhanced by the presence of oxygen, and it is established that as the dose of radiation is increased, the effectiveness of the radiation in destroying target cells is enhanced most dramatically when oxygen is present. Therefore, selectivity for tumor cells toward radiation is difficult to achieve -- normal cells, in view of their oxygen supply, are generally more susceptible to radiation than the target tumor cells. It is therefore desirable to provide a means of sensitizing tumor cells, but not the surrounding tissue, to radiation treatment. One solution would be to increase the supply of oxygen to these tumor cells. This, however, has proved difficult to do.

Various heterocyclic compounds and in particular those with oxidized nitrogen moieties, have been used to radiosensitize hypoxic tumor cells. Indeed, it has been postulated that the oxidized nitrogen functionality is responsible for this activity. Nitroimidazoles, particularly misonidazole (MIS) and metronidazole have been studied extensively, and MIS is commonly used as a standard in in vitro and in vivo tests for radiosensitizing activity. (See, e.g., Asquith, et al, Radiation Res (1974) 60:108-118; Hall, et al, Brit J Cancer (1978) 37: 567-569; Brown, et al, Radiation Res (1980) 82:171-190; and U.S. patent 4,371,540. The radiosensitizing activities of certain 1-substituted 3(5)-nitro-s-triazoles and of various quinoxaline-1,4-dioxide derivatives have also been disclosed.

In addition, US Serial Nos. 730,761, filed 3 May 1985, and 788,762, filed 18 October 1985 assigned to the same assignee and incorporated by reference disclose a group of radiosensitizers that do not contain oxidized nitrogen -- the substituted benzamides and nicotinamides and their thio analogs. These compounds, nevertheless, are radiosensitizers. It is important to distinguish the ability to sensitize hypoxic cells selectively, for instance, by enhancing their oxygen supply, from another mechanism commonly encountered for "sensitizing" cells: inhibition of the enzyme poly(ADP-ribose)polymerase, which is believed to be essential in the repair of irradiated cells after radiation. This repair mechanism is operative in both hypoxic tumor cells and in normal cells. Hence, administration of "radiosensitizers" which operate according to this latter mechanism does not accomplish the desired purpose of selectively sensitizing the target tumor cells.

A group of compounds which has not previously been suggested for use in either selectively killing hypoxic cells or in radiosensitizing such cells is 3-amino-1,2,4-benzotriazine 1,4-di-N-oxide and related compounds. Related US patents 3,980,779; 3,868,371; and 4,001,410 disclose the preparation of a group of these compounds and their use as anti-microbial agents, particularly by addition of these materials to livestock fodder. US patents 3,991,189 and 3,957,799 disclose derivatives of these compounds bearing substituents on the nitrogen of the 3-amino group. These compounds also have anti-microbial activity.

The present invention provides additional compounds which specifically radiosensitize hypoxic cells in vitro and which, furthermore, are directly cytotoxic to hypoxic cells both in vitro and in vivo. Therefore, administration of these compounds prior to or following radiation treatment of tumors selectively kills the hypoxic (tumor) cells which survive the radiation dose. Both the ability of these compounds to radiosensitize hypoxic cells in vitro and especially their ability to selectively kill hypoxic cells directly are unexpected properties of these compounds.

WO-A-8802366, published 7 April 1988, describes the use of certain 1,2,4-benzotriazine oxides as radiosensitizers and selective cytotoxic agents.

The Journal of Organic Chemistry, vol. 24, No. 6, 1959, J. Jiu et al "Synthesis in the 1,2,4-benzotriazine series", pages 813-814 describes the synthesis of a series of 3-substituted-1,2,4-benzotriazine-1-oxides and some corresponding 3-substituted-1,2,4-benzotriazines for pharmacological evaluation.

US-A-2489359 is concerned with certain novel 7-halogen-3-(mono-and di-substituted amino)-1,2,4-benzotriazine compounds which are described as useful intermediates in the preparation of complex organic compounds including anti-malarial agents and dyes.

US-A-2489352 is concerned with substituted 3-amino-1,2,4-benzotriazine-1-oxides considered to possess potential therapeutic value for use in the treatment of malaria.

Journal of the American Chemical Society, Vol. 76 No. 18, 1954, F.J. Wolf et al, pages 4611-4618 and Vol. 76 No. 13, 1954, F.J. Wolf et al, pages 3551-3553 are both concerned with certain 1,2,4-benzotriazine compounds which are considered to have anti-malarial activity.

FR-A-2322140 is concerned with 3-alkoxy-benzo-1,2,4-triazines and their use as fungicides and bactericides.

EP-A-0001090 is concerned with plant bactericide compositions containing 3-halo-benzotriazine-1-oxides.

Journal of Chromatography, Vol. 131, 1977, M.S.F. Ross, pages 448-452 describes the use of a certain methyl substituted 3-dimethylamino-1,2,4-benzotriazine-1-oxide as a starting material for an anti-rheumatic compound azapropazone.

US-A-3482024 describes 1,2,4-benzotriazines for use in alleviating inflammation, edema, pain, fever and swelling of the joints.

GB-A-1234845 is concerned with phosphoric, phosphonic, thionophosphoric and thionophosphonic acid esters of 3-hydroxybenzotriazine-1-N-oxides and their use as insecticides and acaricides.

DE-A-2404375 describes 1,2,4-benzotriazine-1,4-dioxides substituted in the 3-position by -NH-CO- or =C= groupings for use for the control of microorganisms, as feed additives for animals of commercial value and for the protection of materials.

Int. J. Radiation Oncology Biol. Phys., 12, (7), 1986, pages 1239-1242, E.M. Zeman et al. is a further study on 3-amino-1,2,4-benzotrazine-1,4-dioixde as a bioreductive agent with selective toxicity with hypoxic mammalian cells.

In 35th Ann. Meeting of Radiation Res. Soc. and 7th. Ann. Meeting of North Am. Hyperthermia Group, Feb. 21-26, 1987, Atlanta, US, 1986, page 114, No. FD-4; M. Brown et al. bioreductive cytotoxic activity of 3-amino substituted 1,2,4-benzotriazine-1,4-dioxides is mentioned.

Biochemical Pharmacology, 35, No. 19, October 1, 1986, pages 3417-3420, K.R. Laderoute et al., discusses 3-amino-1,2,4-benzotriazine-1,4-dioxide as a hypoxic cell toxic agent and investigates the major reduction products of this compound.

Radiotherapy and Oncology., 12, (1988), pages 209-218, E.M. Zeman et al. relates to a further study of 3-amino-1,2,4-benzotriazine-1,4-dioxide in the selective killing of hypoxic mammalian cells in vitro.

Tetrahedron, 38, (12), 1982, pages 1793-1796, R.H. Atallah et al. describes the preparation of 3-methyl-1,2,4-benzotriazine oxides.

### Disclosure of the Invention

The invention provides a valuable addition to the group of compounds currently available as selective radiosensitizers and selective cytotoxic agents for hypoxic tumor cells. Some of the compounds useful in this regard are known compounds, others are novel.

According to one aspect of the invention, there is provided use of a compound of the formula 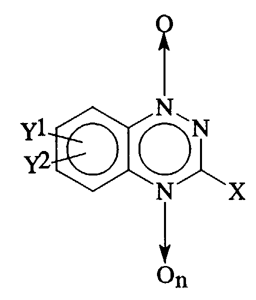 wherein X is H, or hydrocarbyl (1-4C), and said hydrocarbyl may further be optionally substituted with hydroxy, alkoxy, amino, or halogeno;
n is 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1 to 4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage, including hydrocarbyloxy, or
when X is H, methyl or ethyl, one of Y¹ and Y² is H and the other is NH₂
in the manufacture of a medicament for the treatment of the human or animal body for the selective killing of hypoxic tumor cells.

According to another aspect of the invention there is provided a method of selectively killing hypoxic tumor cells in vitro which method comprises administering directly to the cells in vitro a compound of the formula 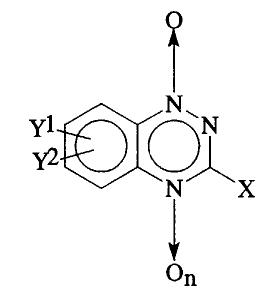 wherein X is H, or hydrocarbyl (1-4C), and said hydrocarbyl may further be optionally substituted with hydroxy, alkoxy, amino, or halogeno;
n is 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1 to 4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy, or
when X is H, methyl or ethyl, one of Y¹ and Y² is H and the other is NH₂.

According to a further aspect of the invention there is provided use of a compound of the formula: 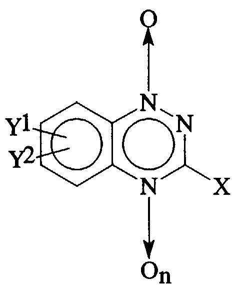 wherein X is H, or hydrocarbyl (1-4C), and said hydrocarbyl may further be optionally substituted with hydroxy, alkoxy, amino, or halogeno;
n is 0 or 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1 to 4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage, including hydrocarbyloxy, or
when n is 1 and X is H, methyl or ethyl, one of Y¹ and Y² is H and the other is NH₂,
in the manufacture of a medicament for the treatment of the human or animal body for the radiosensitising of hypoxic tumor cells.

According to a yet further aspect of the invention, there is provided a method of radiosensitising hypoxic tumor cells in vitro, which method comprises administering a compound of the formula 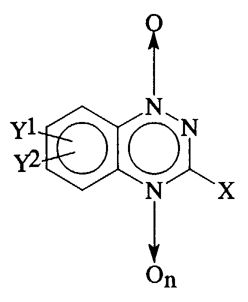 wherein X is H, or hydrocarbyl (1-4C), and said hydrocarbyl may further be optionally substituted with hydroxy, alkoxy, amino, or halogeno;
n is 0 or 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1 to 4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage, including hydrocarbyloxy, or
when n is 1 and X is H, methyl or ethyl, one of Y¹ and Y² is H and the other is NH₂.

The compounds useful according to the invention, therefore, are the mono- or dioxides of optionally substituted 1,2,4-benzotriazine which may contain a hydrocarbyl (1-4C) group, either substituted or unsubstituted, in the 3 position. While all of these compounds are generally effective as radiosensitizers, only compounds which are di-N-oxides (n=1) are effective cytotoxic agents.

Certain of the compounds encompassed by the above Formula are already known in the art as being useful for other purposes; other compounds are novel. The novel compounds encompassed by the present invention and which may be prepared by methods disclosed herein include compounds represented by the formula wherein X is H, or hydrocarbyl (1-4C), and said hydrocarbyl may further be optionally substituted with hydroxy, alkoxy, amino or halogeno;
n is 0 or 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1-4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C), including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; with the provisos that when Y¹ and Y² are hydrogen, X is other than methyl, and when n is 0, at least one of X, Y¹ and Y² is other than hydrogen.

The invention further provides a compound of the formula: 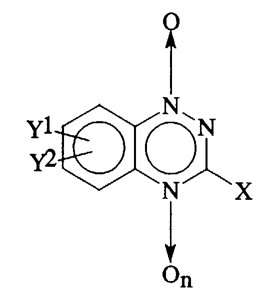 wherein X is H, or hydrocarbyl (1-4C), and said hydrocarbyl may further be optionally substituted with hydroxy, alkoxy, amino or halogeno;
n is 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1-4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C), including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage, including hydrocarbyloxy; with the proviso that when Y¹ and Y² are hydrogen, X is other than methyl for use in the treatment of the human or animal body for the selective killing of hypoxic tumor cells.

### Modes of Carrying Out the Invention

### A. The Compounds Useful in the Invention

The compounds useful in radiosensitizing hypoxic tumor cells as described herein are derivatives of 1,2,4-benzotriazine oxide.

The hydrocarbyl group represented by Y¹ or Y² may contain 1-14 carbon atoms, may be saturated or unsaturated, cyclic or acyclic, and may optionally be interrupted by a single ether linkage. Thus, the unsubstituted form of Y¹ or Y² can be, for example, methyl, ethyl, n-propyl, s-butyl, n-hexyl, 2-methyl-n-pentyl, 2-ethoxyethyl, 3-(n-propoxy)-n-propyl, 4-methoxybutyl, cyclohexyl, tetrahydrofurfuryl, furfuryl, cyclohexenyl, 3-(n-decyloxy)-n-propyl, 4-methyloctyl, 4.7-dimethyloctyl, and the like.

The hydrocarbyl may be substituted with one or two substituents as follows: The halogeno substituents are fluoro, chloro, bromo, or iodo. The alkoxy substituents represented by OR" may contain 1 to 4 carbon atoms, and include, for example, methoxy, n-propoxy, and t-butoxy. The amino substituent may be NH₂, NHR^{a} or NR^{a} ₂, where each R^{a} is independently an alkyl of 1-4 carbons or a morpholino moiety. R^{a} may optionally be substituted with 1-2 hydroxy, alkoxy, amino, or halogeno substituents.

The acyloxy and acylamido groups are represented by R"COO- and R"CONH-, respectively, where R" contains 1-4 carbons, and their thio analogs are represented by R"CSO- and R"CSNH-. Alkyl sulfonyl and alkyl phosphonyl are, respectively, R"SO₂ and R"P(OR")O- wherein each R" is independently as above defined. Carboxy is the group -C(O)OH; alkoxycarbonyl is -C(O)OR"; carbamyl is -C(O)NH₂; and alkylcarbamyl is -C(O)NHR".

Compounds having an amino substituent on the hydrocarbyl side chain can be converted to salts.

The 1,2,4-benzotriazine may be used as the mono- or dioxide. Either the 1-nitrogen of the triazino ring may be oxidized, or both the 1- and 4-nitrogens may be oxidized.

Specific particularly preferred compounds which are useful in the radiosensitization and cytotoxic procedures of the invention include
1,2,4-benzotriazine 1-oxide;
1,2,4-benzotriazine 1,4-dioxide;
3-methyl-1,2,4-benzotriazine 1,4-dioxide;
3-ethyl-1,2,4-benzotriazine 1,4-dioxide;
3-propyl-1,2,4-benzotriazine 1,4-dioxide;
6(7)-amino-1,2,4-benzotriazine 1,4-dioxide;
6(7)-amino-3-methyl-1,2,4-benzotriazine 1,4-dioxide;
6(7)-amino-3-ethyl-1,2,4-benzotriazine 1,4-dioxide;
6(7)-methoxy-1,2,4-benzotriazine 1,4-dioxide;
6(7)-methoxy-3-methyl-1,2,4-benzotriazine 1,4-dioxide;
6(7)-[1-(2,3-dihydroxypropoxy)]-1,2,4-benzotriazine 1,4-dioxide;
6(7)-[1,2-dihydroxyethyl]-1,2,4-benzotriazine 1,4-dioxide;
6(7)-[1-(3-ethylamino-2-hydroxypropoxy)]-1,2,4-benzotriazine 1,4-dioxide;
6(7)-[2-ethylamino-1-hydroxyethyl]-1,2,4-benzotriazine 1,4-dioxide;
6(7)-chloro-1,2,4-benzotriazine 1,4-dioxide;
6(7)-[2-hydroxyethyl]-1,2,4-benzotriazine 1,4-dioxide;
6(7)-[1-hydroxyethyl]-1,2,4-benzotriazine 1,4-dioxide;
and their pharmaceutically acceptable salts and the thioamide analogs of the foregoing list of compounds. It should be noted that the "Y¹ or Y²" substituents set forth in most of the above compounds as present in either the 6 or 7 positions (designated "6(7)") may also be present at the 5 and/or 8 ring positions.

Of the above compounds useful in the method of the present invention as selective cytotoxic agents or radiosensitizers, the following compounds are novel: compounds given by the formula above wherein X is H, or hydrocarbyl (1-4C), and said hydrocarbyl may further be optionally substituted with hydroxy, alkoxy, amino or halogeno;
n is 0 or 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1-4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C), including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; with the provisos that when Y¹ and Y² are hydrogen, X is other than methyl, and when n is 0, at least one of X, Y¹ and Y² is other than hydrogen.

### B. Preparation of the Compounds of the Invention

The dioxide may be prepared from the parent monoxide or 1,2,4-benzotriazine by peracid oxidation (see Robbins et al, J Chem Soc 3186 (1957) and Mason et al, J Chem Soc B 911 (1970)).

In addition, the monoxide may be prepared by:
(1) cyclization of a 1-nitro-2-aminobenzene compounds using H₂NCN;
(2) oxidation of the parent compound given by the structure 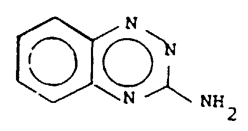 or by controlled reduction of the corresponding dioxide (see Mason, supra, and Wolf et al, J Am Chem Soc 76:355 (1954)).

The 1,2,4-benzotriazines may be prepared by cyclization of formazan precursors using BF₃/AcOH (see Scheme I and Atallah and Nazer, Tetrahedron 38:1793 (1982)).

### C. Formulation and Administration

As demonstrated below, the oxidized benzotriazines of the invention may be used to radiosensitize or selectively kill hypoxic tumor cells in warm-blooded animal hosts. A way in which they may be used is in conjunction with agents known to selectively create hypoxia in tumors. Such methods include the use of antihypertensive drugs such as hydralazine, or agents which affect the amount of oxygen carried by the blood. While these compounds will typically be used in cancer therapy of human patients, they may be used to kill hypoxic tumor cells in other warm blooded animal species such as other primates, farm animals such as cattle, and sports animals and pets such as horses, dogs, and cats.

Hypoxia is believed to be associated with all types of solid malignant neoplasms. The compounds of the invention may, therefore, be used to radiosensitize or to kill neoplastic epithelial cells, endothelial cells, connective tissue cells, bone cells, muscle cells, nerve cells, and brain cells. Examples of carcinomas and sarcomas include carcinomas such as epithelial cell, acidic cell, alveolar cell, basal cell, basal squamous cell, cervical, renal, liver, Hurthle, Lucke, mucinous and Walker, and sarcomas such as Abernathy's, alveolar soft part, angiolithic, botyroid, encephaloid, endometria stroma, Ewing's fascicular, giant cell, lymphatic, Jensen's, juxtacortical osteogenic, Kaposi's, medullary, and synovial. Specific examples of tumors that have been sensitized with other radiosensitizers are reported in Adams, G.E., Cancer: A Comprehensive Treatise (F. Becker, Ed) vol 6, pp 181-223, Plenum, New York, 1977.

The compounds may be administered to patients orally or parenterally (intravenously, subcutaneously, intramuscularly, intraspinally, intraperitoneally, and the like). When administered parenterally the compounds will normally be formulated in a unit dosage injectable form (solution, suspension, emulsion) with a pharmaceutically acceptable vehicle. Such vehicles are typically nontoxic and nontherapeutic. Examples of such vehicles are water, aqueous vehicles such as saline, Ringer's solution, dextrose solution, and Hank's solution and nonaqueous vehicles such as fixed oils (e.g., corn, cottonseed, peanut, and sesame), ethyl oleate, and isopropyl myristate. Sterile saline is a preferred vehicle and the compounds are sufficiently water soluble to provide a solution for all foreseeabie needs. The vehicle may contain minor amounts of additives such as substances that enhance solubility, isotonicity, and chemical stability, e.g., antioxidants, buffers, and preservatives. When administered orally (or rectally) the compounds will usually be formulated into a unit dosage form such as a tablet, capsule, suppository or cachet. Such formulations typically include a solid, semisolid or liquid carrier or diluent. Exemplary diluents and vehicles are lactose, dextrose, sucrose, sorbitol, mannitol, starches, gum acacia, calcium phosphate, mineral oil, cocoa butter, oil of theobroma, aginates, tragacanth, gelatin, syrup, methylcellulose, polyoxyethylene sorbitan monolaurate, methyl hydroxybenzoate, propyl hydroxybenzoate, talc, and magnesium stearate.

The amount of compound administered to the subject is sufficient to radiosensitize or to produce cytotoxicity in the malignant neoplasm to be treated but below that which may elicit toxic effects. This amount will depend upon the type of tumor, the species of the subject being treated, the indication dosage intended and the weight or body surface of the subject. The radiation may be administered to humans in a variety of different fractionation regimes, i.e., the total radiation dose is given in portions over a period of several days to several weeks. These are most likely to vary from daily (i.e., five times per week) doses for up to six weeks, to once weekly doses for four to six weeks. An individual dose of the benzotriazine will be given before or after each radiation treatment and is likely to be in the range of 0.01 to 20 mmol/kg and usually in the range of 0.1 to 2 mmol/kg.

For use as selective cytotoxic agents, the compounds of the invention can be administered alone, with radiation or other cancer cytotoxic agents, with vasoactive drugs (e.g., hydralazine), or with procedures which reduce the amount of available oxygen carried by the blood such as anemia or drugs which increase the binding of oxygen to hemoglobin, all of which can enhance selectively the degree of hypoxia in the tumor. As noted above, while all of the compounds encompassed by the above Formula are generally useful as radiosensitizers herein, only those compounds which are 3-substituted-1,2,4-benzotriazine 1,4-dioxides (n is 1) are useful as selective cytotoxic agents.

### Examples

The following examples further illustrate the compounds of the invention and methods for synthesizing and using them, and are not intended to limit the invention in any manner.

### Example 1: Preparation of 1,2,4-Benzotriazine 1,4-Dioxide

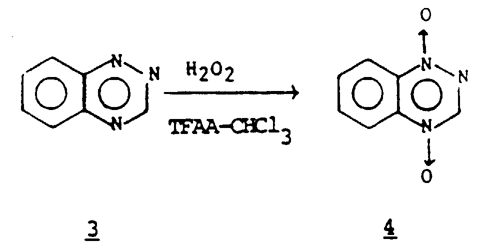

A mixture of 1.80 g (13.73 mmole) of 3, 90% H₂O₂ (9 ml), trifluoroacetic anhydride (13.5 ml) and Na₂WO₄.2H₂O (12.50g, 38 mmole) in CHCl₃ (170 ml) was stirred at room temperature for 5 days. The reaction mixture was diluted with H₂O (100 ml) and extracted with CHCl₃ (100 ml). The organic layer was washed with H₂O (50 ml), dried (Na₂SO₄), and the solvent removed in vacuo. The residue was chromatographed on silica gel using EtOAc-CH₂Cl₂ (1:1) to give 0.30 g (13.4%) of compound 4 as a yellow solid, m.p. 204-205°C. Anal. Calcd. for C₇H₅N₃O₂ (163.13): C, 51.5; H, 3.09; N, 25.76. Found: C, 51.6; H, 3.36; N, 26.01. Mass Spec. M⁺=163 (q=100), 147 (q=50). TLC: R_{f}=0.27 (EtOAc-CH₂Cl₂, 1:1, silica gel plates). IR (nujol): 1600µ, 1460µ, 1300µ, 1230µ. UVₘₐₓ (H₂O): 227 (ε 22,900) 252 (ε 12,950); 392 (ε 4,080).

### Example 2: Determination of LD₅₀

LD₅₀ is determined in BALB/c female mice (weighing 20-25 g) following intraperitoneal (ip) injection, unless the compound tested has low lipophilicity and is very soluble, wherein intravenous (iv) administration is used. LD₅₀ values at 1, 2, 5, and 60 days are determined by administering graded doses of the drug dissolved in physiological saline immediately prior to injection.

## Claims

1. Use of a compound of the formula 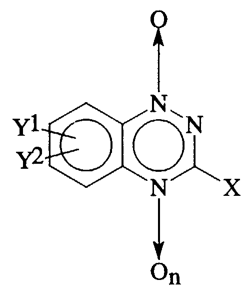 wherein X is H, or hydrocarbyl (1-4C), and said hydrocarbyl may further be optionally substituted with hydroxy, alkoxy, amino, or halogeno;
n is 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1 to 4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage, including hydrocarbyloxy, or
when X is H, methyl or ethyl, one of Y¹ and Y² is H and the other is NH₂
in the manufacture of a medicament for the treatment of the human or animal body for the selective killing of hypoxic tumor cells.

2. Use of
3-ethyl-1,2,4-benzotriazine-1,4-dioxide, or
3-propyl-1,2,4-benzotriazine-1,4-dioxide in the manufacture of a medicament for the treatment of the human or animal body for the selective killing of hypoxic tumor cells.

3. A method of selectively killing hypoxic tumor cells in vitro which method comprises administering directly to the cells in vitro a compound of the formula 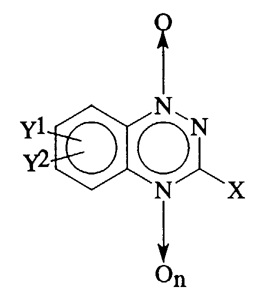 wherein X is H, or hydrocarbyl (1-4C), and said hydrocarbyl may further be optionally substituted with hydroxy, alkoxy, amino, or halogeno;
n is 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1 to 4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy, or
when X is H, methyl or ethyl, one of Y¹ and Y² is H and the other is NH₂.

4. A method of selectively killing hypoxic tumor cells in vitro which method comprises administering directly to the cells in vitro
3-ethyl-1,2,4-benzotriazine-1,4-dioxide, or
3-propyl-1,2,4-benzotriazine-1,4-dioxide.

5. Use of a compound of the formula: 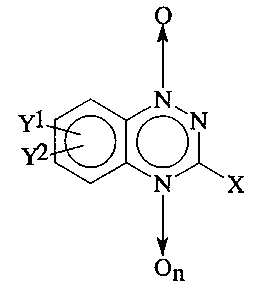 wherein X is H, or hydrocarbyl (1-4C), and said hydrocarbyl may further be optionally substituted with hydroxy, alkoxy, amino, or halogeno;
n is 0 or 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1 to 4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage, including hydrocarbyloxy, or
when n is 1 and X is H, methyl or ethyl, one of Y¹ and Y² is H and the other is NH₂
in the manufacture of a medicament for the treatment of the human or animal body for the radiosensitising of hypoxic tumor cells.

6. Use of a compound selected from the group consisting of:
3-ethyl-1,2,4-benzotriazine-1,4-dioxide, and
3-propyl-1,2,4-benzotriazine-1,4-dioxide in the manufacture of a medicament for the treatment of the human or animal body for the radiosensitising of hypoxic tumor cells.

7. A method of radiosensitising hypoxic tumor cells in vitro, which method comprises administering a compound of the formula 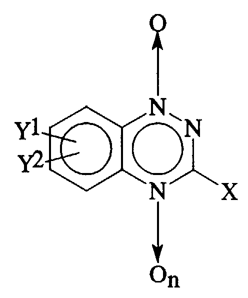 wherein X is H, or hydrocarbyl (1-4C), and said hydrocarbyl may further be optionally substituted with hydroxy, alkoxy, amino, or halogeno;
n is 0 or 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1 to 4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage, including hydrocarbyloxy, or
when n is 1 and X is H, methyl or ethyl, one of Y¹ and Y² is H and the other is NH₂.

8. A method of radiosensitising hypoxic tumor cells in vitro, which method comprises administering a compound selected from the group
3-ethyl-1,2,4-benzotriazine-1,4-dioxide, and
3-propyl-1,2,4-benzotriazine-1,4-dioxide.

9. A compound of the formula: 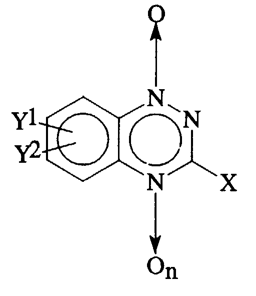 wherein X is H, or hydrocarbyl (1-4C), and said hydrocarbyl may further be optionally substituted with hydroxy, alkoxy, amino or halogeno;
n is 0 or 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1-4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C), including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; with the provisos that when Y¹ and Y² are hydrogen, X is other than methyl, and when n is 0, at least one of X, Y¹ and Y² is other than hydrogen.

10. A compound selected from the group consisting of
3-ethyl-1,2,4-benzotriazine-1,4-dioxide, and
3-propyl-1,2,4-benzotriazine-1,4-dioxide.

11. A compound of the formula: 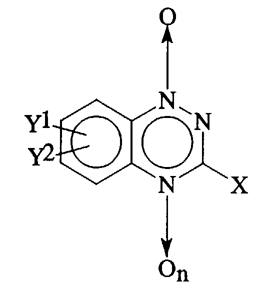 wherein X is H, or hydrocarbyl (1-4C), and said hydrocarbyl may further be optionally substituted with hydroxy, alkoxy, amino or halogeno;
n is 1; and
Y¹ and Y² are independently
H;
halogeno;
hydrocarbyl (1-14C) including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is independently an alkyl of 1-4 carbon atoms or morpholino moiety, said alkyl and morpholino moieties being optionally substituted with one or two substituents selected from hydroxy, alkoxy, amino or halogeno substituents), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage including hydrocarbyloxy; or
NHR', O(CO)R', NH(CO)R', O(SO)R', or O(POR')R' in which R' is hydrocarbyl (1-14C), including cyclic and unsaturated hydrocarbyl, optionally substituted with 1 or 2 substituents selected from the group consisting of halogeno, hydroxy, epoxy, alkoxy, alkylthio, NH₂, NHR^{a}, NR^{a} ₂ (wherein each R^{a} is as defined above), morpholino, acyloxy, acylamido and their thio analogs, carboxy, alkoxycarbonyl, carbamyl or alkylcarbamyl, and wherein the hydrocarbyl can optionally be interrupted by a single ether (-0-) linkage, including hydrocarbyloxy; with the proviso that when Y¹ and Y² are hydrogen, X is other than methyl for use in the treatment of the human or animal body for the selective killing of hypoxic tumor cells.

12. A compound as defined in claim 9 or 10 for use in the treatment of the human or animal body for the radiosensitising of hypoxic tumor cells.

## Patentansprüche

1. Verwendung einer Verbindung der Formel 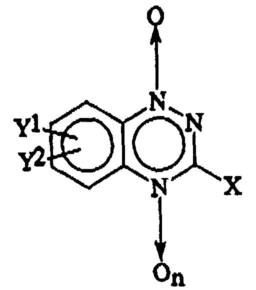 worin X H oder Hydrocarbyl (1-4 C) ist und das Hydrocarbyl ferner wahlweise mit Hydroxy, Alkoxy, Amino oder Halogen substituiert sein kann;
n 1 ist; und
Y¹ und Y² unabhängig
H;
Halogen;
Hydrocarbyl (1-14 C) sind, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} unabhängig ein Alkyl mit 1 bis 4 Kohlenstoffatomen oder eine Morpholinogruppierung ist, wobei die Alkyl- und Morpholinogruppierungen wahlweise mit einem oder zwei Substituenten substituiert sind, gewählt aus Hydroxy-, Alkoxy, Amino- oder Halogensubstituienten), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfung unterbrochen sein kann, einschließlich Hydrocarbyloxy; oder
NHR', O(CO)R', NH(CO)R', O(SO)R' oder O(POR')R', worin R' Hydrocarbyl (1-14 C) ist, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} wie oben definiert ist), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfungen unterbrochen sein kann, einschließlich Hydrocarbyloxy; oder
wenn X H, Methyl oder Ethyl ist, eines von Y¹ und Y² H und das andere NH₂ ist,
bei der Herstellung eines Medikaments zur Behandlung des menschlichen oder tierischen Körpers zur selektiven Abtötung von hypoxischen Tumorzellen.

2. Verwendung von
3-Ethyl-1,2,4-benzotriazin-1,4-dioxid oder
3-Propyl-1,2,4-benzotriazin-1,4-dioxid bei der Herstellung eines Medikaments zur Behandlung des menschlichen oder tierischen Körpers zur selektiven Abtötung von hypoxischen Tumorzellen.

3. Verfahren zum selektiven Abtöten von hypoxischen Tumorzellen in vitro, wobei das Verfahren das direkte Verabreichen an Zellen in vitro einer Verbindung der Formel 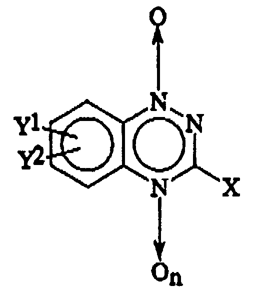 umfaßt, worin X H oder Hydrocarbyl (1-4 C) ist und das Hydrocarbyl ferner wahlweise mit Hydroxy, Alkoxy, Amino oder Halogen substituiert sein kann;
n 1 ist; und
Y¹ und Y² unabhängig
H;
Halogen;
Hydrocarbyl (1-14 C) sind, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} unabhängig ein Alkyl mit 1 bis 4 Kohlenstoffatomen oder eine Morpholinogruppierung ist, wobei die Alkyl- und Morpholinogruppierungen wahlweise mit einem oder zwei Substituenten substituiert sind, gewählt aus Hydroxy-, Alkoxy, Amino- oder Halogensubstituienten), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfung unterbrochen sein kann, einschließlich Hydrocarbyloxy; oder
NHR', O(CO)R', NH(CO)R', O(SO)R' oder O(POR')R', worin R' Hydrocarbyl (1-14 C) ist, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} wie oben definiert ist), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfung unterbrochen sein kann, einschließlich Hydrocarbyloxy; oder
wenn X H, Methyl oder Ethyl ist, eines von Y¹ und Y² H und das andere NH₂ ist.

4. Verfahren zum selektiven Abtöten von hypoxischen Tumorzellen in vitro, wobei das Verfahren das direkte Verabreichen an Zellen in vitro von
3 -Ethyl-1,2,4-benzotriazin-1,4-dioxid oder
3 -Propyl-1,2,4-benzotriazin-1,4-dioxid umfaßt.

5. Verwendung einer Verbindung der Formel: 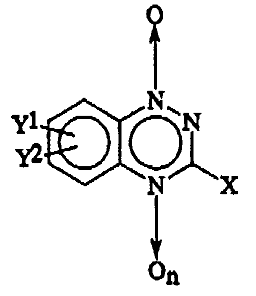 worin X H oder Hydrocarbyl (1-4 C) ist und das Hydrocarbyl ferner wahlweise mit Hydroxy, Alkoxy, Amino oder Halogen substituiert sein kann;
n 0 oder 1 ist; und
Y¹ und Y² unabhängig
H;
Halogen;
Hydrocarbyl (1-14 C) sind, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} unabhängig ein Alkyl mit 1 bis 4 Kohlenstoffatomen oder eine Morpholinogruppierung ist, wobei die Alkyl- und Morpholinogruppierungen wahlweise mit einem oder zwei Substituenten substituiert sind, gewählt aus Hydroxy-, Alkoxy, Amino- oder Halogensubstituienten), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfung unterbrochen sein kann, einschließlich Hydrocarbyloxy; oder
NHR', O(CO)R', NH(CO)R', O(SO)R' oder O(POR')R', worin R' Hydrocarbyl (1-14 C) ist, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} wie oben definiert ist), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfung unterbrochen sein kann, einschließlich Hydrocarbyloxy; oder
wenn n 1 ist und X H, Methyl oder Ethyl ist, eines von Y¹ und Y² H und das andere NH₂ ist,
bei der Herstellung eines Medikaments zur Behandlung des menschlichen oder tierischen Körpers für die Strahlensensibilisierung von hypoxischen Tumorzellen.

6. Verwendung einer Verbindung, gewählt aus der Gruppe bestehend aus:
3-Ethyl-1,2,4-benzotriazin-1,4-dioxid oder
3-Propyl-1,2,4-benzotriazin-1,4-dioxid,
bei der Herstellung eines Medikaments zur Behandlung des menschlichen oder tierischen Körpers für die Strahlensensibilisierung von hypoxischen Tumorzellen.

7. Verfahren zur Strahlensensibilisierung von hypoxischen Tumorzellen in vitro, wobei das Verfahren das Verabreichen einer Verbindung der Formel: 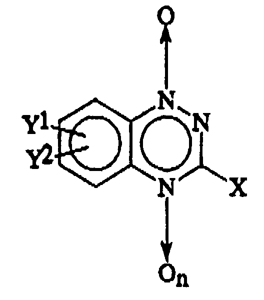 umfaßt, worin X H oder Hydrocarbyl (1-4 C) ist und das Hydrocarbyl ferner wahlweise mit Hydroxy, Alkoxy, Amino oder Halogen substituiert sein kann;
n 0 oder 1 ist; und
Y¹ und Y² unabhängig
H;
Halogen;
Hydrocarbyl (1-14 C) sind, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} unabhängig ein Alkyl mit 1 bis 4 Kohlenstoffatomen oder eine Morpholinogruppierung ist, wobei die Alkyl- und Morpholinogruppierungen wahlweise mit einem oder zwei Substituenten substituiert sind, gewählt aus Hydroxy-, Alkoxy, Amino- oder Halogensubstituienten), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfung unterbrochen sein kann, einschließlich Hydrocarbyloxy; oder
NHR', O(CO)R', NH(CO)R', O(SO)R' oder O(POR')R', worin R' Hydrocarbyl (1-14 C) ist, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} wie oben definiert ist), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfung unterbrochen sein kann, einschließlich Hydrocarbyloxy; oder
wenn n 1 ist und X H, Methyl oder Ethyl ist, eines von Y¹ und Y² H und das andere NH₂ ist.

8. Verfahren zur Strahlensensibilisierung von hypoxischen Tumorzellen in vitro, wobei das Verfahren das Verabreichen einer Verbindung umfaßt, die aus der Gruppe
3-Ethyl-1,2,4-benzotriazin-1,4-dioxid und
3-Propyl-1,2,4-benzotriazin-1,4-dioxid gewählt wird.

9. Verbindung der Formel: 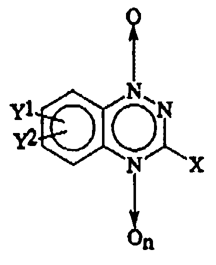 worin X H oder Hydrocarbyl (1-4 C) ist und das Hydrocarbyl ferner wahlweise mit Hydroxy, Alkoxy, Amino oder Halogen substituiert sein kann;
n 0 oder 1 ist; und
Y¹ und Y² unabhängig
H;
Halogen;
Hydrocarbyl (1-14 C) sind, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} unabhängig ein Alkyl mit 1 bis 4 Kohlenstoffatomen oder eine Morpholinogruppierung ist, wobei die Alkyl- und Morpholinogruppierungen wahlweise mit einem oder zwei Substituenten substituiert sind, gewählt aus Hydroxy-, Alkoxy, Amino- oder Halogensubstituienten), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfung unterbrochen sein kann, einschließlich Hydrocarbyloxy; oder
NHR', O(CO)R', NH(CO)R', O(SO)R' oder O(POR')R', worin R' Hydrocarbyl (1-14 C) ist, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} wie oben definiert ist), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfung unterbrochen sein kann, einschließlich Hydrocarbyloxy; mit der Maßgabe, daß wenn Y' und Y² Wasserstoff sind, X etwas anderes als Methyl ist, und wenn n 0 ist, mindestens eines von X, Y¹ und Y² etwas anderes als Wasserstoff ist.

10. Verbindung, gewählt aus der Gruppe bestehend aus:
3-Ethyl-1,2,4-benzotriazin-1,4-dioxid und
3-Propyl-1,2,4-benzotriazin-1,4-dioxid.

11. Verbindung der Formel 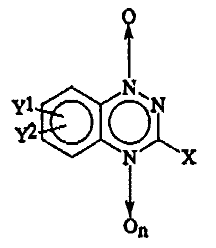 worin X H oder Hydrocarbyl (1-4 C) ist und das Hydrocarbyl ferner wahlweise mit Hydroxy, Alkoxy, Amino oder Halogen substituiert sein kann;
n 1 ist; und
Y¹ und Y² unabhängig
H;
Halogen;
Hydrocarbyl (1-14 C) sind, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} unabhängig ein Alkyl mit 1 bis 4 Kohlenstoffatomen oder eine Morpholinogruppierung ist, wobei die Alkyl- und Morpholinogruppierungen wahlweise mit einem oder zwei Substituenten substituiert sind, gewählt aus Hydroxy-, Alkoxy, Amino- oder Halogensubstituienten), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfung unterbrochen sein kann, einschließlich Hydrocarbyloxy; oder
NHR', O(CO)R', NH(CO)R', O(SO)R' oder O(POR')R', worin R' Hydrocarbyl (1-14 C) ist, einschließlich cyclischem und ungesättigtem Hydrocarbyl, wahlweise substituiert mit 1 oder 2 Substituenten, gewählt aus der Gruppe, bestehend aus Halogen, Hydroxy, Epoxy, Alkoxy, Alkylthio, NH₂, NHR^{a}, NR^{a} ₂ (worin jedes R^{a} wie oben definiert ist), Morpholino, Acyloxy, Acylamido und deren Thioanaloga, Carboxy, Alkoxycarbonyl, Carbamyl oder Alkylcarbamyl, und worin das Hydrocarbyl wahlweise durch eine einzelne Ether (-O-)-Verknüpfungen unterbrochen sein kann, einschließlich Hydrocarbyloxy; mit der Maßgabe, daß wenn Y¹ und Y² Wasserstoff sind, X etwas anderes als Methyl ist, zur Verwendung bei der Behandlung des menschlichen oder tierischen Körpers zum selektiven Abtöten von hypoxischen Tumorzellen.

12. Verbindung gemäß Anspruch 9 oder 10 zur Verwendung bei der Behandlung des menschlichen oder tierischen Körpers zur Strahlensensibilisierung von hypoxischen Tumorzellen.

## Revendications

1. Utilisation d'un composé de formule : 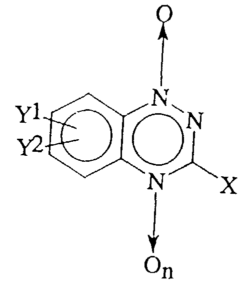 dans laquelle X est H, ou hydrocarbyle (C1-C4), et ledit hydrocarbyle pourront en outre être éventuellement substitué par un hydroxy, alkoxy amine, ou halogéne ;
n est 1, et
Y₁ et Y₂ sont indépendamment H, halogéne, hydrocarbyle (C1-C14), comprenant un hydrocarbyle cyclique et insaturé éventuellement substitué par 1 ou 2 sustituants choisis dans le groupe constitué par un halogéne, hydroxy, époxy, alkoxy, alkylthio, NH₂, NHR^{a}, NR^{a} ₂ (dans lesquels chaque R^{a} est indépendamment un alkyle de 1 à 4 atomes de carbone ou un reste morpholino, lesdits restes alkyle et morpholino étant éventuellement substitués par un ou deux substituants choisis parmi les substituants hydroxy, alkoxy, amino ou halogéne), morpholino, acyloxy, acylamido et leurs analogues thio, carboxy, alkoxycarbonyle, carbarmyle ou alkylcarbamyle, et où l'hydrocarbyle peut éventuellement être interrompu par une liaison éther simple (-0-) comprenant un hydrocarbyloxy ; ou
NHR', O(CO)R', NH(CO)R', O(SO)R', ou O(POR')R' dans lesquels R' est un hydrocarbyle (C1-C14) comprenant un hydrocarbyle cyclique et insaturé, éventuellement substitué par 1 ou 2 substituants choisis dans le groupe constitué par un halogéne, hydroxy, époxy, alkoxy, alkylthio, NH₂, NHR^{a}, NR^{a} ₂ (dans lesquels R^{a} est tel que défini ci-dessus), morpholino, acyloxy, acylamido et leurs analogues thio, carboxy, alkoxycarbonyle, carbamyle ou alkylcarbamyle, et où l'hydrocarbyle peut éventuellement être interrompu par une liaison éther simple (-0-) comprenant un hydrocarbyloxy, ou
lorsque X est H, méthyle ou éthyle, un des radicaux Y¹ et Y² est H et l'autre est NH₂,
dans la préparation d'un médicament pour le traitement du corps humain ou animal en vue de la destruction sélective des cellules tumorales hypoxémiques.

2. Utilisation du 3-éthyle-1,2,4-benzotriazine-1,4-dioxide, ou du 3-propyle-1,2,4-benzotriazine-1,4-dioxide dans la préparation d'un médicament pour le traitement du corps humain ou animal pour la destruction sélective des cellules tumorales hypoxémiques.

3. Procédé de destruction sélective des cellules tumorales hypoxémiques in vitro qui consiste à administrer directement à des cellules in vitro un composé de formule : 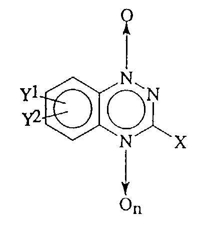 dans laquelle X est H, ou hydrocarbyle (C1-C4), et ledit hydrocarbyle pouvant en outre être éventuellement substitué par un hydroxy, alkoxy amino, ou halogéne ;
n est 1, et
Y¹ et Y² sont indépendamment H, halogéne, hydrocarbyle (C1-C14), comprenant un hydrocarbyle cyclique et insaturé éventuellement substitué par 1 ou 2 sustituants choisis dans le groupe constitué par un halogéne, hydroxy, époxy, alkoxy, alkylthio, NH₂, NHR^{a}, NR^{a} ₂ (dans lesquels chaque R^{a} est indépendamment un alkyle de 1 à 4 atomes de carbone ou un reste morpholino, lesdits restes alkyle et morpholino étant éventuellement substitués par un ou deux substituants choisis parmi les substituants hydroxy, alkoxy, amino ou halogéne), morpholino, acyloxy, acylamido et leurs analogues thio, carboxy, alkoxycarbonyle, carbarmyle ou alkylcarbamyle, et où l'hydrocarbyle peut éventuellement être interrompu par une liaison éther simple (-0-) comprenant un hydrocarbyloxy ; ou
NHR', O(CO)R', NH(CO)R', O(SO)R', ou O(POR')R' dans lesquels R' est un hydrocarbyle (C1-C14) comprenant un hydrocarbyle cyclique et insaturé, éventuellement substitué par 1 ou 2 substituants choisis dans le groupe constitué par un halogéne, hydroxy, époxy, alkoxy, alkylthio, NH₂, NHR^{a}, NR^{a} ₂ (dans lesquels R^{a} est tel que défini ci-dessus), morpholino, acyloxy, acylamido et leurs analogues thio, carboxy, alkoxycarbonyle, carbamyle ou alkylcarbamyle, et où l'hydrocarbyle peut éventuellement être interrompu par une liaison éther simple (-0-) comprenant un hydrocarbyloxy, ou
lorsque X est H, méthyle ou éthyle, un des radicaux Y¹ et Y² est H et l'autre est NH₂,

4. Procédé de destruction sélective de cellules tumorales hypoxémiques in vitro qui consiste à administrer directement aux cellules in vitro du 3-éthyle-1,2,4-benzotriazine-1,4-dioxide, ou du 3-propyle-1,2,4-benzotriazine-1,4-dioxide.

5. Utilisation d'un composé de formule : 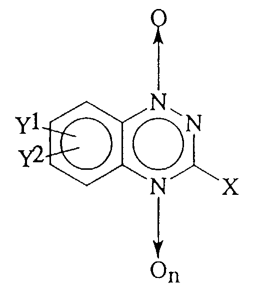 dans laquelle X est H, ou hydrocarbyle (C1-C4), et ledit hydrocarbyle peut en outre être éventuellement substitué par un hydroxy, alkoxy amine, ou halogéne ;
n est 1, et
Y¹ et Y² sont indépendamment H, halogéne, hydrocarbyle (C1-C14), comprenant un hydrocarbyle cyclique et insaturé éventuellement substitué avec 1 ou 2 sustituants choisis dans le groupe constitué par un halogéne, hydroxy, époxy, alkoxy, alkylthio, NH₂, NHR^{a}, NR^{a} ₂ (dans lesquels chaque R^{a} est indépendamment un alkyle de 1 à 4 atomes de carbone ou un reste morpholino, lesdits restes alkyle et morpholino étant éventuellement substitué par un ou deux substituants choisis parmi les substituants hydroxy, alkoxy, amino ou halogéne), morpholino, acyloxy, acylamido et leurs analogues thio, carboxy, alkoxycarbonyle, carbarmyle ou alkylcarbamyle, et où l'hydrocarbyle peut éventuellement être interrompu par une liaison éther simple (-0-) comprenant un hydrocarbyloxy ; ou
NHR', O(CO)R', NH(CO)R', O(SO)R', ou O(POR')R' dans lesquels R' est un hydrocarbyle (C1-C14) comprenant un hydrocarbyle cyclique et insaturé, éventuellement substitué par 1 ou 2 substituants choisis dans le groupe constitué par un halogéne, hydroxy, époxy, alkoxy, alkylthio, NH₂, NHR^{a}, Nr^{a} ₂ (dans lesquels R^{a} est tel que défini ci-dessus), morpholino, acyloxy, acylamido et leurs analogues thio, carboxy, alkoxycarbonyle, carbamyle ou alkylcarbamyle, et où l'hydrocarbyle peut éventuellement être interrompu par une liaison éther simple (-0-) comprenant un hydrocarbyloxy, ou
lorsque X est H, méthyle ou éthyle, un des radicaux Y¹ et Y² est H et l'autre est NH₂,
dans la préparation d'un médicament pour le traitement du corps humain ou animal pour rendre radiosensibles les cellules tumorales hypoxémiques.

6. Utilisation d'un composé choisi dans le groupe constitué par :
le 3-éthyle-1,2,4-benzotriazine-1,4-dioxide, ou le 3-propyle-1,2,4-benzotriazine-1,4-dioxide dans la préparation d'un médicament pour le traitement du corps humain ou animal pour rendre radiosensibles les cellules tumorales hypoxémiques.

7. Procédé pour rendre radiosensibles les cellules tumorales hypoxémiques in vitro qui comprend l'administration d'un composé de formule : 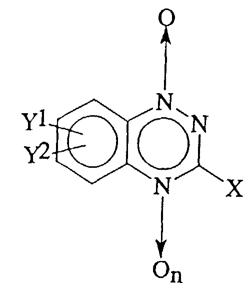 dans laquelle X est H, ou hydrocarbyle (C1-C4), et ledit hydrocarbyle pouvant en outre être éventuellement substitué par un hydroxy, alkoxy amino, ou halogéne ;
n est 1, et
Y¹ et Y² sont indépendamment H, halogéne, hydrocarbyle (C1-C14), comprenant un hydrocarbyle cyclique et insaturé éventuellement substitué avec 1 ou 2 sustituants choisis dans le groupe constitué par un halogéne, hydroxy, époxy, alkoxy, alkylthio, NH₂, NHR^{a}, NR^{a} ₂ (dans lesquels chaque R^{a} est indépendamment un alkyle de 1 à 4 atomes de carbone ou un reste morpholino, lesdits restes alkyle et morpholino étant éventuellement substitués avec un ou deux substituants choisis parmi les substituants hydroxy, alkoxy, amino ou halogéno), morpholino, acyloxy, acylamido et leurs analogues thio, carboxy, alkoxycarbonyle, carbarmyle ou alkylcarbamyle, et dans laquelle l'hydrocarbyle peut éventuellement être interrompu par une liaison éther simple (-0-) comprenant un hydrocarbyloxy ; ou
NHR', O(CO)R', NH(CO)R', O(SO)R', ou O(POR')R' dans lesquels R' est un hydrocarbyle (C1-C14) comprenant un hydrocarbyle cyclique et insaturé, éventuellement substitué par 1 ou 2 substituants choisis dans le groupe constitué par un halogéne, hydroxy, époxy, alkoxy, alkylthio, NH₂, NHR^{a}, NR^{a} ₂ (dans lesquels R^{a} est tel que défini ci-dessus), morpholino, acyloxy, acylamido et leurs analogues thio, carboxy, alkoxycarbonyle, carbamyle ou alkylcarbamyle, et où l'hydrocarbyle peut éventuellement être interrompu par une liaison éther simple (-0-) comprenant un hydrocarbyloxy, ou
lorsque X est H, méthyle ou éthyle, un des radicaux Y¹ et Y² est H et l'autre est NH₂,

8. Procédé pour rendre radiosensibles des cellules tumorales hypoxémiques in vitro qui comprend l'administration d'un composé choisi dans le groupe constitué par le 3-éthyle-1,2,4-benzotriazine-1,4-dioxide, ou le 3-propyle-1,2,4-benzotriazine-1,4-dioxide.

9. Composé de formule : 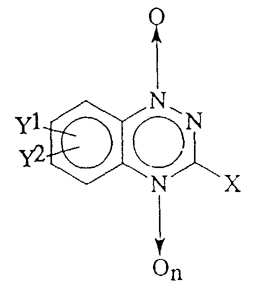 dans laquelle X est H, ou hydrocarbyle (C1-C4), et ledit hydrocarbyle pouvant en outre être éventuellement substitué par un hydroxy, alkoxy amino, ou halogène,
n est 0 ou 1, et
Y¹ et Y² sont indépendamment H, halogéne, hydrocarbyle (C1-C14), comprenant un hydrocarbyle cyclique et insaturé éventuellement substitué par 1 ou 2 sustituants choisis dans le groupe constitué par un halogéne, hydroxy, époxy, alkoxy, alkylthio, NH₂, NHR^{a}, NR^{a} ₂ (dans lesquels chaque R^{a} est indépendamment un alkyle de 1 à 4 atomes de carbone ou un reste morpholino, lesdits restes alkyle et morpholino étant éventuellement substitués par un ou deux substituants choisis parmi les substituants hydroxy, alkoxy, amino ou halogéne), morpholino, acyloxy, acylamido et leurs analogues thio, carboxy, alkoxycarbonyle, carbarmyle ou alkylcarbamyle, et où l'hydrocarbyle peut éventuellement être interrompu par une liaison éther simple (-0-) comprenant un hydrocarbyloxy ; ou
NHR', O (CO)R', NH(CO)R', O(SO)R', ou O(POR')R' dans lesquels R' est un hydrocarbyle (C1-C14) comprenant un hydrocarbyle cyclique et insaturé, éventuellement substitué par 1 ou 2 substituants choisis dans le groupe constitué par un halogéne, hydroxy, époxy, alkoxy, alkylthio, NH₂, NHR^{a}, NR^{a} ₂ (dans lesquels R^{a} est tel que défini ci-dessus), morpholino, acyloxy, acylamido et leurs analogues thio, carboxy, alkoxycarbonyle, carbamyle ou alkylcarbamyle, et où l'hydrocarbyle peut éventuellement être interrompu par une liaison éther simple (-0-) comprenant un hydrocarbyloxy ; mais sous réserve que lorsque Y¹ et Y² sont l'hydrogène, X est différent de méthyle et que lorsque n est 0, au moins l'un de X, Y¹ e Y² est différent de l'hydrogène.

10. Composé choisi dans le groupe constitué par le 3-éthyle-1,2,4-benzotriazine-1,4-dioxide, ou le 3-propyle-1,2,4-benzotriazine-1,4-dioxide.

11. Composé de formule : 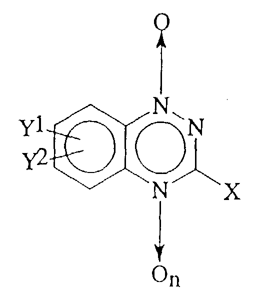 dans laquelle X est H, ou hydrocarbyle (C1-C4), et ledit hydrocarbyle pouvant en outre être éventuellement substitué par un hydroxy, alkoxy amino, ou halogéne
n est 1, et
Y¹ et Y² sont indépendamment H, halogéne, hydrocarbyle (C1-C14), comprenant un hydrocarbyle cyclique et insaturé éventuellement substitué par 1 ou 2 sustituants choisis dans le groupe constitué par un halogéne, hydroxy, époxy, alkoxy, alkylthio, NH₂, NHR^{a}, NR^{a} ₂ (dans lesquels chaque R^{a} est indépendamment un alkyle de 1 à 4 atomes de carbone ou un reste morpholino, lesdits restes alkyle et morpholino étant éventuellement substitués par un ou deux substituants choisis parmi les substituants hydroxy, alkoxy, amino ou halogéne), morpholino, acyloxy, acylamido et leurs analogues thio, carboxy, alkoxycarbonyle, carbarmyle ou alkylcarbamyle, et où l'hydrocarbyle peut éventuellement être interrompu par une liaison éther simple (-0-) comprenant un hydrocarbyloxy ; ou
NHR', O (CO)R', NH (CO)R', O (SO)R', ou O (POR')R' dans lesquels R' est un hydrocarbyle (C1-C14) comprenant un hydrocarbyle cyclique et insaturé, éventuellement substitué par 1 ou 2 substituants choisis dans le groupe constitué par un halogéne, hydroxy, époxy, alkoxy, alkylthio, NH₂, NHR^{a}, NR^{a} ₂ (dans lesquels R^{a} est tel que défini ci-dessus), morpholino, acyloxy, acylamido et leurs analogues thio, carboxy, alkoxycarbonyle, carbamyle ou alkylcarbamyle, et dans laquelle l'hydrocarbyle peut éventuellement être interrompu par une liaison éther simple (-0-) comprenant un hydrocarbyloxy ; sous réserve que lorsque Y¹ et Y² sont l'hydrogène, X est différent de méthyle, pour son utilisation dans le traitement du corps humain ou animal pour la destruction sélective des cellules tumorales hypoxémiques.

12. Composé tel que défini selon la revendication 9 ou 10, pour son utilisation dans le traitement du corps humain ou animal pour rendre radiosensibles les cellules tumorales hypoxémiques.
